# EUROPEAN PATENT APPLICATION

(11) **EP 3 967 304 A1**
(43) Date of publication of application: **16.03.2022**
(21) Application number: 21196270.9
(22) Date of filing: 13.09.2021
(51) Int. Cl.: A61K 31/198, A23L 33/175, A61Q 1/00

(54) **BALANCED AMINO ACID FORMULATION**

(30) Priority: 14.09.2020 IT 202000021664
(71) Applicant: IAF Network S.p.A., 25125 Brescia (IT)
(72) Inventor: TRAPANI, Gabriele, I-43014 MEDESANO, PARMA (IT)
(74) Representative: Croce, Valeria

(57) **Abstract**

The present invention relates to a balanced formulation based on amino acids, which finds application in the field of functional and antiaging medicine, as well as in the cosmetic field for the well-being of hair, skin and nails.

## Description

### Technical field of the invention

The present invention finds application in the nutraceutical field and, in particular, in clinical nutrition and functional medicine.

### Background art

Preparations based on amino acids as food supplements are known.

The need to supplement nutrition with a surplus of amino acids is typical in periods of strong physical stress, such as the period of recovery from an illness, or in periods of intense sporting activity.

The preparations available today follow a standard approach, which includes the nine essential amino acids, although often formulated with a questionable balance from the physiological viewpoint, which does not lead to optimized protein synthesis processes.

Instead in other cases, the proposed amino acid profile is similar to that of red meat, with the same negative and pro-aging effects of methionine.

### Summary of the invention

The present patent application provides a formulation with an optimal amino acid balance, which overcomes the drawbacks of the known formulations.

### Object of the invention

A first object of the present invention is represented by an amino acid preparation.

In other objects of the present invention, the preparation is described for use in the nutraceutical and cosmetic field and in a method for improving the aesthetic appearance of hair, skin and nails.

### Detailed description of the invention

A first object of the present invention is represented by a preparation comprising the following amino acids:
Leucine
Valine
Isoleucine
Lysine
Phenylalanine
Threonine
Methionine
Histidine
Cysteine
Tryptophan
Tyrosine

In an embodiment of the present invention, no other amino acids are comprised in the preparation of the invention.

In a preferred aspect, within the described formulation, the amino acids leucine, isoleucine and valine are present in a molar ratio of about 2:1:1.

According to an aspect of the present invention, the above-mentioned amino acids are contained in the preparation in an amount of:

| **Amino acid** | **% (g/100g *serving* composition)** |
|---|---|
| Leucine | 30.76 |
| Valine | 15.38 |
| Isoleucine | 15.38 |
| Lysine | 16.92 |
| Phenylalanine | 2.30 |
| Threonine | 8.46 |
| Methionine | 1.53 |
| Histidine | 3.07 |
| Cysteine | 4.61 |
| Tryptophan | 0.76 |
| Tyrosine | 0.76 |

According to a preferred aspect of the present invention, a described preparation comprises:

| **Amino acid** | **amount (mg)** |
|---|---|
| Leucine | 2000 |
| Valine | 1000 |
| Isoleucine | 1000 |
| Lysine | 1100 |
| Phenylalanine | 150 |
| Threonine | 550 |
| Methionine | 100 |
| Histidine | 200 |
| Cysteine | 300 |
| Tryptophan | 50 |
| Tyrosine | 50 |

According to a preferred aspect of the present invention, the amino acids described are all obtained from fermentation of vegetable origin; thereby, the preparation can also be advantageously taken by or administered to vegetarians and vegans.

According to an aspect of the present invention, the described preparation must be taken before and/or after performance, and preferably both before and after, regardless it is a sporting activity or a busy working day.

The assumption is reported in terms of serving, which corresponds to about 100 g of amino acid mixture.

In a preferred aspect, the assumption should occur far from meals to allow a better absorption of the amino acid components.

The preparation of the invention can be formulated in powder form, to be dosed appropriately according to need or it can be in the form of swallowable tablets.

The formulation will possibly have further components such as sweeteners and/or flavorings or any additives to increase the solubility of the preparation.

However, it is not excluded that the described preparation may be reformulated as a component of more complex food preparations, such as milk-based drinks, energy bars, etc.

Therefore, such food preparations comprise the preparation of the invention.

In accordance with other objects of the present invention, the preparation is described for use in the nutraceutical field.

In particular, such a preparation is described for use in clinical nutrition and functional antiaging medicine.

According to a further aspect of the present invention, the described preparation is used in cosmetics.

In particular, the functional preparation of the invention is used for the well-being and tone of skin, for the beauty of hair and for the strength of nails.

According to another object, it is described a method for improving the aesthetic appearance of hair, skin and nails comprising the administration of a preparation of the invention to a subject.

From the above description, the numerous advantages offered by the present invention will be immediately apparent to the person skilled in the art.

In particular, the described preparation allows an optimal balance between branched amino acids and essential or "conditionally essential" amino acids.

Thereby, the drawbacks of the currently known preparations are advantageously overcome, in which the imbalance of the ratio between methionine and cysteine results in a pro-aging effect which, in the long term, can lead to homocysteinemia conditions.

The preparation of the invention is therefore an excellent food supplement tool in view of particularly stressful physiological conditions, such as intense and long workouts, conditions of fasting or recovery from diseases, states of ketosis or unbalanced diets, for example lacking carbohydrates.

Furthermore, by virtue of the composition thereof, the preparation of the invention allows to prevent and combat sarcopenia and to confer or increase the anabolic resistance induced by aging.

As reported above, since the amino acids used are all obtained by fermentation of vegetable origin, the preparation of the invention meets the needs of vegetarians or vegans as well.

## Claims

1. An amino acid preparation comprising amino acids and other components, wherein said amino acids are represented by:
Leucine
Valine
Isoleucine
Lysine
Phenylalanine
Threonine
Methionine
Histidine
Cysteine
Tryptophan
Tyrosine

2. An amino acid preparation according to the preceding claim, wherein the amino acids leucine, isoleucine and valine are present in a molar ratio of 2:1:1.

3. An amino acid preparation according to claim 1 or 2, wherein said amino acids are contained in the following amounts:
| **amino acid** | **% (g/100 g *serving* composition)** |
|---|---|
| Leucine | 30.76 |
| Valine | 15.38 |
| Isoleucine | 15.38 |
| Lysine | 16.92 |
| Phenylalanine | 2.30 |
| Threonine | 8.46 |
| Methionine | 1.53 |
| Histidine | 3.07 |
| Cysteine | 4.61 |
| Tryptophan | 0.76 |
| Tyrosine | 0.76 |

4. An amino acid preparation according to any one of the preceding claims, comprising:
| **amino acid** | **amount (mg)** |
|---|---|
| Leucine | 2000 |
| Valine | 1000 |
| Isoleucine | 1000 |
| Lysine | 1100 |
| Phenylalanine | 150 |
| Threonine | 550 |
| Methionine | 100 |
| Histidine | 200 |
| Cysteine | 300 |
| Tryptophan | 50 |
| Tyrosine | 50 |

5. An amino acid preparation according to any one of the preceding claims, wherein said amino acids are obtained by fermentation of vegetable origin.

6. An amino acid preparation according to any one of the preceding claims, in the form of a powder or in the form of swallowable tablets.

7. A food preparation comprising the amino acid preparation according to any one of claims 1 to 6.

8. The food preparation according to the preceding claim, which is in the form of milk-based drinks or energy bars.

9. Use of the amino acid preparation according to any one of claims 1 to 6 or of the food preparation according to claim 7 in clinical nutrition and functional antiaging medicine.

10. Use of the amino acid preparation according to any one of claims 1 to 6 or of the food preparation according to claim 7 or 8 in cosmetics.
